# EUROPÄISCHE PATENTANMELDUNG

(11) **EP 0 677 588 A2**
(43) Veröffentlichungstag der Anmeldung: **18.10.1995**
(21) Anmeldenummer: 95103370.3
(22) Anmeldetag: 09.03.1995
(51) Int. Cl.: C12Q 1/68, C12Q 1/02

(54) **Testverfahren zur Identifizierung von Substanzen, die auf Endothelinrezeptoren wirken**

(30) Priorität: 15.03.1994 DE 4408690
(71) Anmelder: BASF AKTIENGESELLSCHAFT, D-67056 Ludwigshafen (DE)
(72) Erfinder: Kröger, Burkhard, Dr., D-67117 Limburgerhof (DE); Bialojan, Siegfried, Dr., D-68723 Oftersheim (DE); Bollschweiler, Claus, Dr., D-69118 Heidelberg (DE)

(57) **Zusammenfassung**

Testverfahren zur Identifizierung von Substanzen, die auf Endothelinrezeptoren (ETR) wirken, welches die folgenden Schritte umfaßt:
a) Wahl einer Zellinie, die einen Endothelinrezeptor (ETR) endogen exprimiert oder Herstellen einer Zellinie, die einen ETR rekombinant stabil exprimiert,
b) Transfektion einer Wirtszelle nach Schritt a) mit einem rekombinanten Vektor, der
   b1) einen Promotor enthält, der durch Erhöhung intrazellulärer Botenstoffe aktiviert wird, und
   b2) ein Reportergen, funktionell mit dem in b1) beschriebenen Promotor verknüpft, trägt,
c) Etablieren von Zellen nach Schritt b), die
   c1) den unter Schritt b) beschriebenen Vektor transient enthalten, oder
   c2) den unter Schritt b) beschriebenem Vektor stabil integriert enthalten,
d) Exposition der Zellen aus Schritt c) mit der zu testenden Substanz,
e) Messung der Expression des Reportergens in den Zellen nach Schritt d).

SEQUENZPROTOKOLL

(1) ALLGEMEINE INFORMATION:

(i) ANMELDER:
(A) NAME: BASF Aktiengesellschaft
(B) STRASSE: Carl-Bosch-Strasse 38
(C) ORT: Ludwigshafen
(E) LAND: Bundesrepublik Deutschland
(F) POSTLEITZAHL: D-67056
(G) TELEPHON: 0621/6048526
(H) TELEFAX: 0621/6043123
(I) TELEX: 1762175170

(ii) ANMELDETITEL: Testverfahren zur Identifizierung von Substanzen, die
auf Endothelinrezeptorem wirken.

(iii) ANZAHL DER SEQUENZEN: 4

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 75 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: NEIN

(vi) URSPRÜNLICHE HERKUNFT:
(A) ORGANISMUS: Human Cytomegalo Virus

(viii) POSITION IM GENOM:
(B) KARTENPOSITION: Immediate Early Gene Promotor

(xi) SEQUENZBESCHREIBUNG: SEQ ID NO: 1:

GGCCGCTGAC GCAAATGGGC GGTAGGCGTG TACGGTGGGA GGTCTATATA AGCAGAGCTC 60

GTTTAGTGAA CCGTA 75

(2) INFORMATION ZU SEQ ID NO: 2:

(i) SEQUENZ CHARAKTERISTIKA:
(A) LÄNGE: 75 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Human Cytomegalo Virus

(viii) POSITION IM GENOM:
(B) KARTENPOSITION: Immediate Early Gene Promotor

(xi) SEQUENZBESCHREIBUNG: SEQ ID NO: 2:

CTAGTACGGT TCACTAAACG AGCTCTGCTT ATATAGACCT CCCACCGTAC ACGCCTACCG 60

CCCATTTGCG TCAGC 75

(2) INFORMATION ZU SEQ ID NO: 3:

(i) SEQUENZ CHARAKTERISTIKA:
(A) LÄNGE: 106 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: NEIN

(vi) URSPRÜNLICHE HERKUNFT:
(A) ORGANISMUS: Homo sapiens

(xi) SEQUENZBESCHREIBUNG: SEQ ID NO: 3:

TATGCCCCAT GGCCGTCATA CTGTGACGTC CCCATGGCCG TCATACTGTG ACGTCCCCAT 60

GGCCGTCATA CTGTGACGTC CCCATGGCCG TCATACTGTG ACGTGC 106

(2) INFORMATION ZU SEQ ID NO: 4:

(i) SEQUENZ CHARAKTERISTIKA:
(A) LÄNGE: 108 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Homo sapiens

(xi) SEQUENZBESCHREIBUNG: SEQ ID NO: 4:

GGCCGCACGT CACAGTATGA CGGCCATGGG GACGTCACAG TATGACGGCC ATGGGGACGT 60

CACAGTATGA CGGCCATGGG GACGTCACAG TATGACGGCC ATGGGGCA 108

## Beschreibung

Die vorliegende Erfindung betrifft ein funktionelles Testverfahren zur Identifizierung von Substanzen, die auf Endothelinrezeptoren wirken.

Endotheline (ET) sind Peptide, bestehend aus 21 Aminosäuren, die in Endothelzellen und vielen anderen Geweben als Vorläuferproteine (Prepro-ET, big-ET) exprimiert werden und durch die entsprechenden Endothelin konversionsenzyme (ECEs) in die biologisch aktive Form überführt werden (Inoue, A. et al. Proc.Natl.Acad.Sci.USA, 86, 2863-2867, 1989; Yanagisawa, M. et al. TIPS 10, 374-378, 1989).

Man kennt drei verschiedene Isoformen von Endothelinen, ET-1, ET-2, ET-3, die sich in zwei bzw. fünf Aminosäuren voneinander unterscheiden und auf unterschiedlichen Genen kodiert sind. Endotheline haben einen starken Einfluß auf die Blutdruckregulation (Yanagisawa, M. et al. Nature 332, 411-415, 1988).

Basis dieser Wirkung ist die Interaktion der Endotheline mit spezifischen Rezeptoren (ETR) auf Glattmuskelzellen (ETRa) und Endothelzellen (ETRb), deren Primärstrukturen aufgeklärt sind (Arai, H. et al. Nature 348, 730-732, 1990; Sakurai, T. et al. Nature 348, 732-735, 1990).

ET-Rezeptoren gehören der Familie der 7-Transmembrandomänen-Rezeptoren an, die intrazelluär an G-Proteine gekoppelt sind und dadurch spezifische "second messenger"-Kaskaden in Gang setzen (Calcium, cAMP, PIP, cGMP).

Für eine pharmakologische Beeinflussung des Blutdrucks sind Substanzen von Interesse, die die Wirkung der Endotheline an ihren Rezeptoren beeinflussen. Insbesondere sind ETRa-Amtagonisten von Interesse als Blutdrucksenker; ETRb-Agonisten sind als Vasodilatoren einsetzbar.

Das klassische Screening nach Substanzen mit Wirkung auf Rezeptoren beruht auf Rezeptorbindungsassays. Nach diesem Vorgehen läßt sich allerdings nichts über die Funktion von identifizierten Wirksubstanzen (Antagonismus, Agonismus) aussagen. Diese Information muß erst in komplexeren in vitro-Modellen (z.B. radioaktive cAMP-Bestimmung) bzw. in aufwendigen Tierversuchen erarbeitet werden.

Es bestand daher die Aufgabe, Testsysteme zur Verfügung zu stellen, die es erlauben, geeignete Substanzen mit agonistischen oder antagonistischen Eigenschaften zu Endothelinrezeptoren zu identifizieren.

Diese Aufgabe wurde gelöst durch das erfindungsgemäße Testverfahren zur Identifizierung von Substanzen, die auf Endothelinrezeptoren (ETR) wirken, welches die folgenden Schritte umfaßt:
a) Wahl einer Zellinie, die einen Endothelinrezeptor (ETR) endogen exprimiert oder Herstellen einer Zellinie, die einen ETR rekombinant stabil exprimiert,
b) Transfektion einer Wirtszelle nach Schritt a) mit einem rekombinanten Vektor, der
   b1) einen Promotor enthält, der durch Erhöhung intrazellulärer Botenstoffe aktiviert wird, und
   b2) ein Reportergen, funktionell mit dem in b1) beschriebenen Promotor verknüpft, trägt,
c) Etablieren von Zellen nach Schritt b), die
   c1) den unter Schritt b) beschriebenen Vektor transient enthalten, oder
   c2) den unter Schritt b) beschriebenem Vektor stabil integriert enthalten,
d) Exposition der Zellen aus Schritt c) mit der zu testenden Substanz,
e) Messung der Expression des Reportergens in den Zellen nach Schritt d).

Das Grundprinzip der Erfindung beruht auf der Kopplung der durch Ligandenbindung induzierten Rezeptoraktivierung und der damit verbundenen Induktion der Signaltranduktionskette (z.B. cAMP-Generierung) an ein hochsensitives Reportersystem.

Das Reportersystem besteht aus einem Konstrukt, das einen Promotor enthält, der durch Änderung der Konzentration an intrazellulären Botenstoffen ("second messengers") aktivierbar ist. Vorzugsweise kommen hier Promotoren zum Einsatz, die auf Calcium, cAMP, cGMP oder Phosphoinositolphosphat (PIP) ansprechen.

Diese Promotoren regulieren Gene, deren Genprodukt leicht meßbar ist. Vorzugsweise kommen hierbei Reportergene wie Luziferase, Chloramphenicolacetyltransferase, alkalische Phosphatase, β-Galactosidase zum Einsatz.

Im ersten Schritt a) des erfindungsgemäßen Testverfahrens wird eine Zellinie selektioniert, die einen Endothelin-Rezeptor endogen exprimiert. Dabei kann es sich um den ETRa (z.B. bei SKNMC, Rat1, A10, A7R5) oder um ETRb (Endothelzellen) handeln.

Alternativ wird eine Wirtszelle nach der in Beispiel 3 beschriebenen Methode mit einem rekombinanten Vektor stabil transformiert, der für den entsprechenden Endothelin-Rezeptor (ETRa opder ETRb) kodiert (s. Beispiel 1).

Zweckmäßigerweise kann der rekombinante Vektor noch weitere Genabschnitte tragen, so zum Beispiel Regulationssignale oder Selektionsmarker. Besonders vorteilhaft ist die Verwendung eines Selektionsmarkers wie Neomycin-Resistenz (neoR).

Als Wirtszelle für die Expression eines ETR eignet sich besonders die CHO-K1-Zellinie (ATCC Nr. CCL 61).

In einem weiteren Schritt wird ein rekombinanter Vektor hergestellt, der einen Promotor trägt, welcher abhängig von der Konzentration an intrazellulären Botenstoffen reguliert wird. Unter intrazellulären Botenstoffen oder "second messengers" sind insbesondere Calcium, cAMP, cGMP und Phosphoinositolphosphat zu verstehen.

Unter einem solchen Promotor ist der Sequenzbereich zu verstehen, der alle, für die "second messenger"-kontrollierte Transkription notwendigen Elemente umfaßt. Darunter fallen sowohl Elemente der basalen Transkriptionskontrolle, wie die "TATA-box" und die "CCAAT-box", als auch Elemente der induzierbaren Transkriptionskontrolle, wie Bindungssequenzen für "second messenger"-abhängige Transkriptionsfaktoren, sogenannte "response elements" (REs).

Hierbei kann es sich um Calcium-REs, cAMP-REs, cGMP-REs oder Phosphoinositolphosphat-REs handeln. Die Sequenz dieser REs und die an sie bindenden Faktoren sind bekannt, so z.B. für das cAMP response element binding protein (CREB) (Montminy, M.R. et al., Proc. Natl. Acad. Sci. U.S.A. 83, 6682-6686, 1986).

Die für die Erfindung geeigneten Promotoren können sowohl natürlich vorkommende sein, wie z.B. der Promotor des Somatostatingens, des fos-Gens, des HIV-1 LTRs oder des vasoactive intestinal peptide (VIP)-Gens, oder aus mehreren verschiedenen Genpromotoren künstlich zusammengesetzt sein.

Dies kann beispielsweise über Fusion und Ligation synthetischer Oligonukleotide erfolgen. Bevorzugt wird die Fusion mehrerer cAMP response elements mit dem basalen Cytomegalovirus IE-Genpromotor durchgeführt.

An diesen zusammengestzten Promotor wird ein Reportergen funktionell angeknüpft. Reportergene sind solche Gene, deren exprimiertes Genprodukt ein leicht meß- bzw. quantifizierbares Signal liefert.

In der Regel sind geeignete Reportergene Gene für solche Proteine, die mit gängigen Methoden nachweisbar sind. Bevorzugt werden als Reportergene Gene für Enzyme verwendet, die eine leicht nachweisbare Reaktion katalysieren.

Besonders geeignete Reportergene sind die Gene für Chloramphenicol-Acetyl-Transferase (CAT) oder Luziferase.

Die funktionelle Verknüpfung von zusammengestztem Promotor und Reportergen bedeutet, daß die Transkription des Reportergens unter der Kontrolle des "second messenger"-abhängigen Promotors erfolgt.

Die Transformation einer Wirtszelle nach Schritt a) durch den rekombinanten Vektor (Schritt b)) kann durch alle gängigen Transformationsverfahren, z.B. Elektroporation, Calciumphosphat oder Liposomen vermittelte Transfektion erfolgen. Besonders gut geeignet für die Transformation ist die in Beispiel 3 benützte Liposomen vermittelte Transfektion.

Nachdem die Wirtszelle mit dem rekombinanten Vektor transformiert worden ist, werden in einem weiteren Schritt c) solche Zellen weiterbenutzt, die den Vektor entweder transient oder stabil integriert exprimieren.

Unter transienter Expression versteht man eine Expression von nur begrenzter Dauer (etwa 24 - 48h). Für die stabile Expression integriert der Vektor in das Genom der Wirtszelle.

In einem weiteren Schritt d) werden die im Schritt c) isolierten Zellen den zu testenden Substanzen zusammen mit dem spezifischen ETR-Liganden (z.B. ET-1) ausgesetzt.

Dies geschieht dadurch, daß die zu testenden Substanzen üblicherweise in einer Konzentration von 10⁻³M bis 10⁻⁶M sowie Endothelin (ET-1) üblicherweise in einer Konzentration von 10⁻⁸M dem serumfreien Zellkulturmedium zugesetzt werden.

Die zu testenden Substanzen bleiben in der Regel 2 bis 24 Stunden mit den ET-stimulierten Wirtszellen in Kontakt. Anschließend werden die Zellen lysiert und für die Auswertung der Reportergen-Expression vorbereitet, wie es in Beispiel 4 angegeben ist.

Die Expression des Reportergens in einer mit einer Testsubstanz behandelten Testzelle wird gemessen und verglichen mit einer Kontrolle, bei der die gleiche Zellinie lediglich mit ET stimuliert wurde. Durch Vergleich der beiden Meßwerte läßt sich sofort eine Aussage darüber machen, ob die getestete Substanz die Reportergenexpression und somit die ET-ETR-Interaktion beeinflußt.

Entscheidender Vorteil der vorliegenden Erfindung ist ein Testverfahren, das direkt, mit hoher Sensitivität und hoher Durchsatzrate funktionelle Daten bzgl. Wirkung von Substanzen auf ET-Rezeptoren liefert (Antagonismus, Agonismus). Dadurch wird es ermöglicht, ein effektives Massenscreening nach Substanzen durchzuführen, die auf ET-Rezeptoren wirken.

Die Erfindung wird durch folgende Beispiele weiter veranschaulicht.

### Beispiel 1

### Konstruktion von Vektoren, die für den ETRa bzw. ETRb kodieren.

Die c-DNA des humanen ETa-Rezeptors wurde auf Basis der publizierten Daten (Adachi, M. et al. Biochem. Biophys. Res. Communic. 180, 1265-72, 1991) aus einer humanen Plazenta c-DNA-Bank isoliert und in die multiple cloning site (Hind III / Xho I ) des Plasmids p Bluescript SK (Stratagene, La Jolla ) kloniert. Die kodierende Sequenz für ETRa beträgt 1284 bp.

Die c-DNA des humanen ETb-Rezeptors wurde auf Basis der publizierten Daten (Nakamuta, M. et al., Biochem. Biophys. Res. Communic. 177, 34 -39, 1991) aus einer humanen Leber c-DNA-Bank isoliert und in p Bluescript SK (BamH I/Hind III) kloniert. Die kodierende Sequenz für ETRb beträgt 1329 bp.

Zur rekombinanten Expression wurden die beiden Rezeptoren in den Eukaryontenvektor pcDNA I NEO ( Invitrogen, San Diego ) umkloniert. Die beiden Rezeptor DNA Fragmente wurden hierzu jeweils mit den Restriktionsenzymen BamH I / Xho I aus p Bluescript isoliert und in die entsprechenden Schnittstellen der multiple cloning site von pcDNA I NEO ligiert.

Die zur Durchführung der Arbeiten angewendeten Techniken sind übliche molekularbiologische Techniken, die z. B. in "Molecular Cloning", Sambrook et al, Cold Spring Harbor Laboratory, 1989 beschrieben sind.

### Beispiel 2

### Konstruktion des zusammengesetzten Promotors und Fusion mit dem Reportergen

a) Ausgehend von dem käuflichen Reporterkonstruckt pMAMneo-luc (Fa. Clontech, Kat.No.6171-1) wurde ein Enhancer/Promotorfreies Reporterplasmid konstruiert. Dazu wurde der die regulatorischen Sequenzen des RSV-LTRs und des MMTV-LTRs enthaltende Bereich in pMAMneo-luc durch Restriktionsspaltung mit NdeI und NheI deletiert und durch einen Polylinker, der die Erkennungssequenzen für die Restriktionsenzyme NdeI, MluI, NotI, SpeI und NheI enthält, ersetzt. Das so entstandene Reporterplasmid pneoLuc dient somit der Aufnahme regulatorischer Sequenzen in die Polylinkerregion.
   Der zusammengesetzte Promotor wurde aus vier synthetischen Oligonukleotiden hergestellt:
b) SEQ ID NO: 1 entspricht der Promotorregion des humanen Cytomegalovirus Immediate Early Gens von Position -67 bis +1 (Hennighausen L.G., Fleckenstein B.; EMBO J. 5, 1367-1371, 1986).
   Zusätzlich enthält SEQ ID NO: 1 zwei Erkennungssequenzen für Restriktionsendonukleasen: am 5' Ende für NotI, am 3' Ende für SpeI.
   SEQ ID NO: 2 entspricht dem Gegenstrang zu SEQ ID NO: 1.
c) SEQ ID NO: 3 entspricht der vierfachen Wiederholung des cAMP response elements des vasoactive intestinal peptide (VIP) Gens (Tsukada, T. et al. J.Biol.Chem. 262, 8743-8747, 1987).
   Zusätzlich enthält SEQ ID NO: 3 zwei Erkennungssequenzen für Restriktionsendonukleasen: am 5' Ende für NdeI, am 3' Ende für NotI.
   SEQ ID NO: 4 entspricht dem Gegenstrang zu SEQ ID NO: 3.
d) Zur Herstellung des rekombinanten Vektors wurden SEQ ID NO: 1 und SEQ ID NO: 2 hybridisiert und NotI/SpeI gespalten, sowie SEQ ID NO: 3 und SEQ ID NO: 4 hybridisiert und NdeI/NotI gespalten. Beide Produkte wurden dann in den mit NdeI und SpeI linearisierten Vektor pneoLuc aus Beispiel 2a) kloniert. Diese Klonierungsstrategie hat die richtige, d.h. funktionelle Orientierung der Einzelkomponenten zur Folge.
   Durch Deletion des BglII/HindIII Restriktionsfragments aus pneoLuc wurde das NeoR-Gen so verkürzt, daß es nicht mehr funktionell war. Dies war notwendig, da die zu transfizierenden Wirtszellen bereits über den ETRa, bzw.ETRb-Vektor (S. Beispiel 1) gegen Neomycin resistent waren und somit eine entsprechende Selektion nicht möglich war. Die Selektion nach stabilen Transformanden erfolgte durch eine Kotransfektion mit einem Vektor, der ein Hygromycinresistenzgen trägt (Stratagene) durch Kultivierung in Hygromycin-haltigem Medium (Beispiel 3).

### Beispiel 3

### Transformation und Selektion einer stabilen Testzellinie

CHO-K1-Zellen wurden in DMEM/HamF12-(1:1)-Medium mit 10% FCS in einer Zelldichte von 1x106 Zellen in 10cm-Petrischalen eingesät und über Nacht kultiviert.

Die Transfektion erfolgte mit 2 µg Vektor-DNA in 10 µl Transfectam (Promega Nr. E 1231), aufgenommen in 2 ml serumfreiem Medium über 6 Stunden. Danach erfolgte ein Überführen in serumhaltiges Medium. Nach weiteren 42 Stunden wurden die Zellen in Selektionsmedium überführt.

Im Falle der ETR-exprimierenden Wirtszellen nach Schritt a) erfolgte die Selektion in Medium, das 600 µg/ml Geneticin (G418-Sulfat) enthielt.

Zellen, die den Vektor nach Schritt b) stabil exprimieren, wurden durch Selektion in Medium, dem 50 µg/ml Hygromycin zugesetzt wurde, erhalten.

### Beispiel 4

### Durchführung des Tests

Zellen gemäß Beispiel 3 wurden in Mikrotiterplatten (Packard "Viewplate" Nr 600-5182) eingesät (Zelldichte 1x105 Zellen/well; DMEM/HamF12-Medium mit 10% FCS) und nach 16 Stunden in serumfreies Medium überführt. Nach weiteren 4 h Stunden wurden Verdünnungen der zu testenden Substanzen zusammen mit 10⁻⁸M ET-1 zugegeben. Kontrollzellen wurden nur mit ET-1 behandelt.

Nach 2-16 Stunden Inkubation wurden die Zellen durch Zugabe von 30 æl/well Lysispuffer (25nM Tris-Phosphat, pH 7,8; 2mM DTT; 2mM 1,2-Diaminocyclohexan -N,N,N',N'-Tetraessigsäure; 10% Glycerol; 1% Triton X-100) aufgeschlossen (15 min. Raumtemperatur).

Zu diesem Zellextrakt wurden 50 µl Luziferase Assay Substrate (270 µM Coenzym A, 470 µM Luciferin, 530 µM ATP) zugegeben. Die Messung der Luziferase-Aktivität kann in herkömmlichen Luminometern durchgeführt werden; vorzugsweise erfolgt die Messung in einem 2D-Luminometer der Firma Hamamatsu.

### Beispiel 5

### Einsatz des Testsystems im Massenscreening

Das Assaysystem gemäß Beispiel 4 wird eingesetzt für das "Random Screening" nach Substanzen, die spezifisch die Interaktion von Endothelin mit seinen Rezeptoren beeinflussen.

Substanzen, die im Screening verwendet werden sollen, werden in µM Konzentrationen in DMSO gelöst und in geeigneten Verdünnungen gemäß Beispiel 4 im Test eingesetzt.

## Patentansprüche

1. Testverfahren zur Identifizierung von Substanzen, die auf Endothelinrezeptoren (ETR) wirken, welches die folgenden Schritte umfaßt:
a) Wahl einer Zellinie, die einen Endothelinrezeptor (ETR) endogen exprimiert oder Herstellen einer Zellinie, die einen ETR rekombinant stabil exprimiert,
b) Transfektion einer Wirtszelle nach Schritt a) mit einem rekombinanten Vektor, der
b1) einen Promotor enthält, der durch Erhöhung intrazellulärer Botenstoffe aktiviert wird, und
b2) ein Reportergen, funktionell mit dem in b1) beschriebenen Promotor verknüpft, trägt,
c) Etablieren von Zellen nach Schritt b), die
c1) den unter Schritt b) beschriebenen Vektor transient enthalten, oder
c2) den unter Schritt b) beschriebenem Vektor stabil integriert enthalten,
d) Exposition der Zellen aus Schritt c) mit der zu testenden Substanz,
e) Messung der Expression des Reportergens in den Zellen nach Schritt d).

2. Testverfahren nach Anspruch 1, dadurch gekennzeichnet, daß als Reportergen in Schritt b2) Luziferase verwendet wird.

3. Testverfahren nach Anspruch 1 oder 2, dadurch gekennzeichnet, daß als Zelle in Schritt die Zelle CHO-Kl verwendet wird.
